Europäisches Patentamt

European Patent Office

Office européen des brevets

(19)

(11) Publication number: **0 000 987**
**B1**

(12) **EUROPEAN PATENT SPECIFICATION**

(45) Date of publication of patent specification: **20.01.82**

(51) Int. Cl.³: **A 61 N  1/36**

(21) Application number: **78300241.3**

(22) Date of filing: **03.08.78**

(54) **Demand pacer with programmable rate hysteresis.**

(30) Priority: **19.08.77 GB 3491377**
**19.06.78 US 917141**

(43) Date of publication of application:
**07.03.79 Bulletin 79/5**

(45) Publication of the grant of the European patent:
**20.01.82 Bulletin 82/3**

(84) Designated Contracting States:
**DE FR GB NL SE**

(56) References cited:
**US - A - 3 833 005**
**US - A - 3 999 557**

(73) Proprietor: **BIOTRONIK Mess- und Therapiegeräte**
**GmbH & Co Ingenieurbüro Berlin**
**Sieversufer 8**
**D-1000 Berlin 47 (DE)**

(72) Inventor: **Digby, Dennis**
**2409 Gunflint Trail**
**Brooklyn Park Minnesota 55444 (US)**
Inventor: **Keller, John Walter**
**8600 S.W. 54th Avenue**
**Miami Florida (US)**

(74) Representative: **Christiansen, Henning, Dipl.-Ing.**
**Unter den Eichen 108a**
**D-1000 Berlin 45 (DE)**

Courier Press, Leamington Spa, England.

Demand pacer with programmable rate hysteresis

Technical Field

This invention relates to demand cardiac pacemakers having a programmable rate hysteresis function.

Background Art

Pacemakers for generating artificial stimulating pulses for the heart, and which may be implanted in the body, are well known. Originally the electrical circuitry for such pacemakers was of analog design, but in recent years digital circuitry has been also employed. A digital approach to pacemakers has led to the evolution of programmable pacemakers — pacemakers having parameters such as pulse rates which are adjustable (programmable) once the pacemaker has been implanted. The programs can be changed from outside the patient's body by appropriate signal transmission to the implanted pacemaker and without surgery. Programmable pacemakers are described in, for instance, British Specifications 1,385,954 and 1,398,875. Such pacemakers have circuitry to detect and decode signals transmitted outside the body and alter the program accordingly. In British Specification 1,385,954 (claiming priority based on U.S.S.N. 141,694, in turn a parent of U.S.P.N. 3,805,796 to Tenz) the programming is accomplished by means of a magnetic field which is sensed by a magnetic reed switch; the opening and closing of the switch providing programming pulses to a program store. In British Specification 1,398,875 (based on U.S.P.N. 3,833,005 to Wingrove) the programming is by means of radio frequency transmission and reception.

Many pacemakers are of the demand type — that is they only supply a stimulating pulse to the heart when a natural heart beat is absent. To accomplish this, demand pacemakers have means for sensing the presence or absence of natural heart beats and for actuating the stimulating pulse as appropriate.

It is desirable with a demand pacemaker that the stimulating pulses are issued only when really needed by the heart, and that the latter is given the opportunity of functioning as naturally as possible. One approach to providing this desirable property has been to provide the implanted pacemaker with a fixed hysteresis function for the pacing rate, so that, after each natural heart beat detected which inhibits a stimulating pulse, a slight delay occurs before the next stimulating pulse is generated. Hysteresis is therefore the characteristic of a pacemaker whereby the period of time from a natural heart beat to the next pacing pulse is longer than the period between two successive pacing pulses. This hysteresis, which essentially involves the pacemaker switching over to issuing one stimulating pulse at a slower rate after one or more natural beats has arisen, is of particular use in that it avoids competition between natural heart beats and artificial stimulating pulses, and hence reduces current drain on the pacemaker. It is desirable to allow as many natural beats to arise normally without any stimulation of the heart being provided: by supplying a hysteresis function, a greater opportunity is being given for the natural beats to continue without an artificial pulse being generated.

Not all cardiac specialists agree that a hysteresis function is universally desirable for all pacemakers (see for example, The American Journal of Cardiology, *38*, p. 685—688 (1976)), and with currently available pacemakers this entails a decision on the medical personnel whether or not a pacemaker to be implanted should be one with or without hysteresis in its circuitry. To change this decision entails replacing the implanted pacemaker and hence surgery.

A pacemaker with an optional hysteresis function has been described in U.S. Patent 3,999,557. This pacemaker has different standby (monitoring) and pacing rates (giving a rate hysteresis function) and circuitry for selectively rendering these rates identical (giving a normal demand pacemaker function). These rates are dictated by the charging and discharging of two capacitors under the control of a memory store in the form of a flip-flop. The latter may be remotely programmed (e.g. when the pacemaker is implanted) by the closing of a reed switch. The pacemaker as described represents an analog approach to the control of the pulse rates and does not suggest the possibility of selecting one of a plurality of differently programmable rate hysteresis functions.

Disclosure of Invention

We have now designed an implantable demand cardiac pacemaker, whereby hysteresis can be included in or removed from the functioning circuitry implanted in the Patient's body, which is based upon a digital approach to the selection of pacemaker rates. This not only provides good flexibility and accuracy, but also, if desired, enables a plurality of different hysteresis functions to be built into the pacemaker whereby any of these may be programmed and selected after implant, according to the patient's condition.

According to the invention there is provided a demand cardiac pacemaker having a rate hysteresis function which may be programmably stored therein from a remote programming means so that it optionally operates in said mode when desired, comprising:

(a) an oscillator (1) having a fixed predetermined rate,

(b) an output amplifier (6) for generating artificial stimulating pulses, .

(c) an input amplifier (11) for sensing naturally occurring heartbeat signals,

(d) means including a memory store (13) programmable from the remote programming means for enabling the pacemaker to operate in its rate hysteresis mode when desired, characterised in that the pacemaker comprises:

(e) counting means (2) responsive to said oscillator (1) for supplying output pulses at at least first and second count rates (Qy, Qx),

(f) switching means (3, 4) alternatively responsive to said count rates for energising the output amplifier (6), and

(g) logic means (7, 15) responsive to said memory store (13) and said input amplifier (11) for controlling the switching means (3, 4) whereby, when the memory store (13) dictates that a rate hysteresis mode is not desired, the output amplifier (6) receives counts at said first count rate and, when the memory store (13) dictates that a rate hysteresis mode is desired, the output amplifier (6) receives at least one pulse at said second count rate upon sensing by the logic means of at least one natural heartbeat.

Brief Description of the Drawings

Preferred features of the invention are illustrated in the accompanying drawings, in which:

Figure 1 shows schematically the electrical circuit diagram of an implantable, demand, cardiac pacemaker in which the hysteresis function is programmable;

Figure 2 is an electrical timing diagram for use with Figure 1; and

Figure 3 illustrates an alternative embodiment of the invention which may be employed with Figure 1.

Best Mode for Carrying Out the Invention

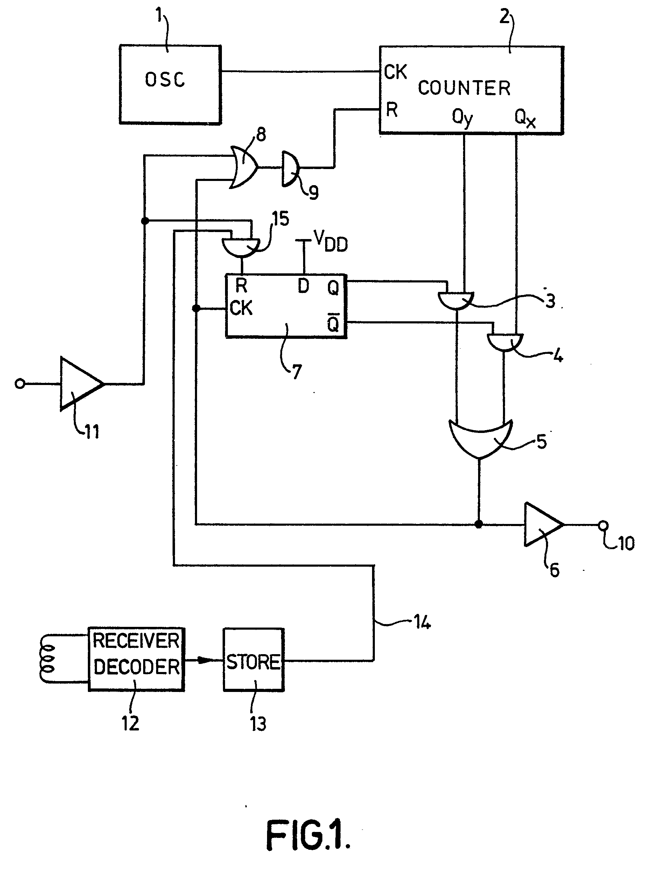

Referring to Figure 1, the pacemaker comprises an oscillator 1 which clocks a counter 2. The counter provides two outputs, Qx which can be considered as issuing pulses at "slow" tissue stimulation pulse rate (e.g. 60 pulses per minute), and Qy which can be considered as issuing pulses at a "normal" tissue stimulation pulse rate (e.g. 70 pulses per minute).

The Qy and Qx outputs supply an input, respectively, to AND gates 3 and 4, whose outputs are provided to an OR gate 5. The output of the latter is supplied to an output amplifier 6, to the clock input of a D-flip-flop 7, and to an OR gate 8. The latter output supplies a delay 9, the output of which is connected to the reset of counter 2.

The output amplifier 6 provides amplified tissue stimulating pulses to a connection 10 for coupling to an electrode leading to the heart.

An input amplifier 11 receives electrical signals detected at the heart (e.g. arising from a natural heart beat) and supplies these to a second input of OR gate 8.

A receiver/decoder 12 is arranged to receive and decode data signals transmitted from outside the patient's body to the implanted pacemaker, and to employ the decoded signals for changing a pacemaker program held in program store 13. For the purposes of illustration, the receiver/decoder 12 and store 13 have been depicted very simply and as providing an output for controlling only the hysteresis function. In practice it would be desirable to make these features much more sophisticated so that the program store is employed to provide a varying control for several different pacemaker parameters (e.g. pulse rate, pulse width, and varying amounts — of hysteresis with various programs, depending upon past history of spontaneous beats). The data signals may be transmitted to the receiver/decoder 12 by any suitable means, but preferably we employ data signals transmitted by tone burst modulation (a carrier frequency being pulse width modulated). In the illustrated embodiment, the output supplied by program store 13 is a single "bit" of binary information, which is provided on line 14 to an AND gate 15. A second input to AND gate 15 is supplied from input amplifier 11. The output of AND gate 15 is connected to the reset of flip-flop 7. The D-input to flip-flop 7 is supplied from the positive supply rail. The Q and $\overline{Q}$ outputs of flip-flop 7 are supplied as inputs to AND gates 3 and 4, respectively.

The pacemaker functions as follows. Assume initially that the hysteresis function has not been selected. This will be provided by storing a "0" in store 13 so as to prevent any reset for flip-flop 7 via line 14 and AND gate 15 occurring. Assume that, when switched on initially, the counter 2 has issued a count via OR gate 5, and that flip-flop 7 is in the "1" state with its Q output high.

The high Q output of flip-flop 7 enables AND gate 3 and, correspondingly, the low $\overline{Q}$ output disables AND gate 4. In this circumstance, OR gate 5 transmits, to the output amplifier 6, the Qy or "normal" pulse rate provided by counter 2.

In the absence of a natural heart beat being detected and amplified by input amplifier 11, oscillator 1 clocks counter 2 and the Qy "normal" pulses are transmitted to the heart via output amplifier 6 and connection 10. Each issued pulse resets counter 2 via OR gate 8 and delay 9 so that the counter commences its count for the next "normal" pulse. The delay provided by delay 9 sets the pulse width for each pulse issued by counter 2.

If a natural heart beat is detected and amplified by amplifier 11 then this signal resets counter 2 via OR gate 8 and delay 9. This reset, unless it occurs just as counter 2 issues a pulse, prevents counter 2 from generating stimulating pulses and no artificial stimulation is provided to the heart. It is of no consequence if the reset initiated by a natural heart beat, arrives just as an artificial pulse is generated, since the natural beat and the stimulating pulse will essentially coincide.

The circuit thus far described is acting as a conventional demand pacemaker, only issuing tissue stimulating pulses for output to the heart when a natural beat is missing.

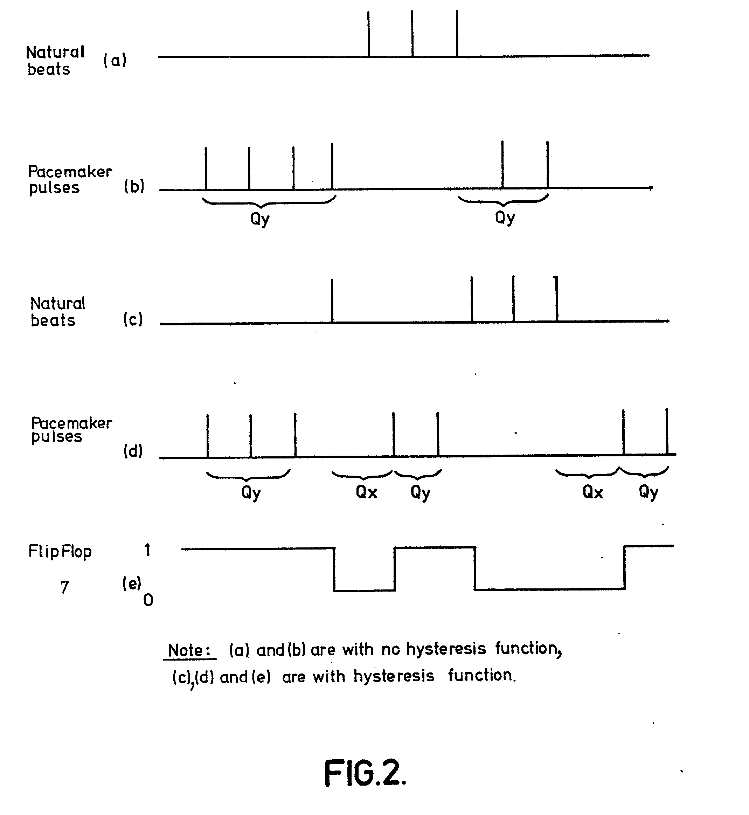

The pulses received by input amplifier 11 and transmitted as artificial stimulating pulses, by output amplifier 6 are illustrated in Figure 2 (a) and (b) respectively.

Assume now that it is desired to select the hysteresis function. In this circumstance a "1" is stored in program store 13 so that a "1" is permanently held on the input line 14 to AND gate 15. In the absence of a natural heart beat, no input is again detected by amplifier 11 and the pacemaker issues "normal" rate tissue stimulating pulses as described above.

If a single natural beat is issued by the heart, this is detected and amplified by amplifier 11 and the signal not only resets counter 2 as described above, but also resets flip-flop 7. The latter thus goes to the "0" state, consequentially disabling AND gate 3 and enabling AND gate 4. This enables the Qx "slow" pulse rate to be steered through OR gate 5 rather than the Qy "normal" rate.

This circumstance causes the next artificial pulse to be issued by counter 2 at a later time than would have been expected at the Qy "normal" rate: it is issued at the Qx "slow" rate. This slight delay provides the hysteresis function for the pacemaker and allows more time for a natural heart beat to be detected and amplified by amplifier 11, so as to inhibit this next artificial pulse. Since we are assuming that only a single natural heart beat arises, no such further natural beat is detected and the Qx "slow" pulse is generated and supplied to the heart. This pulse resets counter 2 via OR gate 8 and delay 9 and also clocks flip-flop 7 so that the latter reverts to the "1" state. This causes a changeover of the states of AND gates 3 and 4 so that there is a reversion to the Qy "normal" pulse rate until the next natural beat resets flip-flop 7 again.

If a succession of natural beats arise, then each will not only reset counter 2 but hold flip-flop 7 reset. The latter holds in the "0" state ready to steer a Qx "slow" pulse to the output once the natural beats decrease in period to below the Qx rate.

The hysteresis function is illustrated by Figure 2 (c), (d) and (e). With no natural beats arising, artificial pulses are issued at the Qy "normal" rate, but when a natural beat occurs, the next artificial pulse is issued at the Qx "slow" rate. After one "slow" pulse the pacemaker reverts to issuing "normal" pulses until the next natural beat arises. As illustrated the next natural beat is one of a succession of three natural beats and no artificial pulse is issued. At the end of the natural beats, one Qx "slow" pulse is issued before the pacemaker again reverts to its "normal" rate.

As has been mentioned above, in practice program store 13 would have the added capability of altering, inter alia, the "normal" pulse rate. This may be accomplished by expanding the outputs obtained from counter 2, and increasing the number of AND and OR gates 3, 4, and 5, and supplying the outputs of the OR gates to a rate decoder which selects, under control from program store 13, the "normal" rate to be generated.

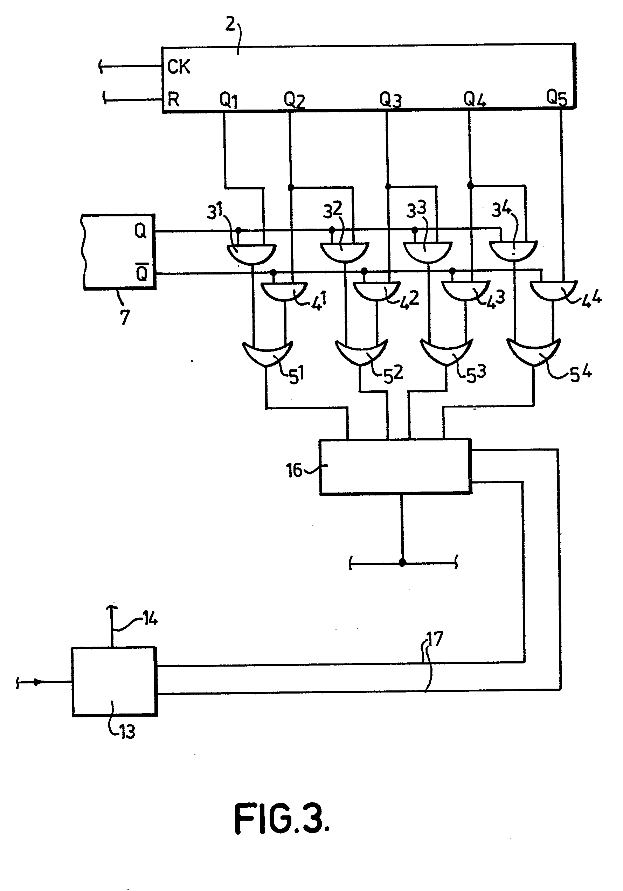

An example of this is indicated in Figure 3 where the number of outputs from counter 2 has been expanded to 5 ($Q_1$ to $Q_5$). Figure 3 is identical to Figure 1 except as indicated. In Figure 3, a plurality of AND gates 3 and 4 ($3^1$ to $3^4$, $4^1$ to $4^4$) and OR gates 5 ($5^1$ to $5^4$) exist. For any pair of adjacent outputs from counter 2, the lower numbered stage can be considered as providing the "normal" pulse rate and the adjacent higher numbered stage as providing the "slow" pulse rate (i.e. $Q_1$ normal, $Q_2$ slow; $Q_2$ normal, $Q_3$ slow . . .). This provides a total of four normal/slow pulse rate combinations which can be individually selected by a rate decoder 16. The particular rate combination selected is determined by the logic levels on lines 17 held in store 13.

## Claims

1. A demand cardiac pacemaker having a rate hysteresis function which may be programmably stored therein from a remote programming means so that it optionally operates in said mode when desired, comprising:

(a) an oscillator (1) having a fixed predetermined rate,

(b) an output amplifier (6) for generating artificial stimulating pulses,

(c) an input amplifier (11) for sensing naturally occurring heartbeat signals,

(d) means including a memory store (13) programmable from the remote programming means for enabling the pacemaker to operate in its rate hysteresis mode when desired, characterised in that the pacemaker comprises:

(e) counting means (2) responsive to said oscillator (1) for supplying output pulses at at least first and second count rates (Qy, Qx),

(f) switching means (3, 4) alternatively responsive to said count rates for energising the output amplifier (6) and

(g) logic means (7, 15) responsive to said memory store (13) and said input amplifier (11) for controlling the switching means (3, 4) whereby, when the memory store (13) dictates that a rate hysteresis mode is not desired, the output amplifier (6) receives counts at said first count rate and, when the memory store (13) dictates that a rate hysteresis mode is desired, the output amplifier (6) receives at least one pulse at said second count rate upon sensing by the logic means of at least one natural heartbeat.

2. A demand cardiac pacemaker according to claim 1, characterised in that said logic means comprises a flip-flop (7) having first and second logic states controlling said switching means (3, 4) to enable the output amplifier (11) to receive counter pulses at said first or said second rate, respectively.

3. A demand cardiac pacemaker according to claim 2 characterised in that said switching means includes first (3) and second (4) gating means respectively receiving output pulses at said first and second rates, said gating means being enabled in complementary fashion by said first and second logic states of said flip-flop (7).

4. A demand cardiac pacemaker according to claim 3, characterised in that said logic means comprises

first means including means (8, 9) responsive to an artificial stimulating pulse or to a naturally occurring heartbeat signal for resetting said counting means (2) to a datum state and means (CK on 7) responsive to an artificial stimulating pulse for setting said flip-flop (7) to enable said first gating means (3), and

second means (15) responsive to said memory store (13), in the event that a rate hysteresis function is desired, and to a naturally occurring heartbeat signal for resetting said flip-flop (7) to enable said second gating means (4).

5. A demand cardiac pacemaker according to any of claims 1 to 4, characterised in that said counting means (2) is adapted to supply output pulses at more than first and second count rates, said additional rates corresponding to respective predetermined stimulating pulse rates, said memory store (13) additionally comprises means (17) for selecting one of said additional rates, and said switching means includes means $(3^1$—$3^4$, $4^1$—$4^4$, $5^1$—$5^4$, 16) responsive to said memory store (13) and to said logic means for supplying pulses at any given one of the pulse rates to said output amplifier (6).

**Revendications**

1. Stimulateur d'impulsions cardiaque à la demande ayant une fonction d'hystérésis de vitesse qui peut y être mémorisée de manière programmable à distance à partir d'un moyen de programmation, si bien qu'il fonctionne de manière optionnelle dans ledit mode lorsque cela est souhaité, comprenant:

(a) un oscillateur (1) ayant un taux prédéterminé fixe,

(b) un amplificateur de sortie (6) destiné à produire des impulsions de stimulation artificielles,

(c) un amplificateur d'entrée (11) destiné à détecter l'apparition naturelle de signaux de battement cardiaque,

(d) un moyen comportant une mémoire (13) programmable à distance à partir du moyen de programmation afin d'autoriser le stimulateur d'impulsions à fonctionner dans son mode d'hystérésis de vitesse lorsque cela est souhaité, caractérisé en ce que le stimulateur d'impulsions comprend:

(e) un moyen de comptage (2) qui répond audit oscillateur (1) en délivrant des impulsions de sortie au moins à un premier et à un deuxième taux de comptage (Qy, Qx),

(f) un moyen de commutation (3, 4) qui répond alternativement auxdits taux de comptage en excitant l'amplificateur de sortie (6), et

(g) un moyen logique (7, 15) qui répond à ladite mémoire (13) et audit amplificateur d'entrée (11) en commandant le moyen de commutation (3, 4) de manière que, lorsque la mémoire (13) décrète qu'un mode d'hystérésis de vitesse n'est pas souhaité, l'amplificateur de sortie (6) reçoive des valeurs de comptage audit premier taux de comptage et, lorsque la mémoire (13) décrète qu'un mode d'hystérésis de vitesse est souhaité, l'amplificateur de sortie (6) reçoive au moins une impulsion audit deuxième taux de comptage après détection par le moyen logique d'au moins un battement cardiaque naturel.

2. Stimulateur d'impulsions cardiaque à la demande selon la revendication 1, caractérisé en ce que ledit moyen logique comprend une bascule (7) ayant un premier et un deuxième état logique commandant ledit moyen de commutation (3, 4) de façon à autoriser l'amplificateur de sortie (11) à recevoir des impulsions du compteur respectivement audit premier taux ou audit deuxième taux.

3. Stimulateur d'impulsions cardiaque à la demande selon la revendication 2, caractérisé en ce que ledit moyen de commutation comporte un premier (3) et un deuxième (4) moyen logique de type porte recevant respectivement des impulsions de sortie audit premier et audit deuxième taux, les moyens logiques du type porte étant validés de façon complémentaire par ledit premier et ledit deuxième état logique de ladite bascule (7).

4. Stimulateur d'impulsions cardiaque à la demande selon la revendication 3, caractérisé en ce que ledit moyen logique comprend un premier moyen comportant un moyen (8, 9) qui répond à une impulsion de stimulation artificielle ou à un signal de battement cardiaque survenant naturellement en repositionnant ledit moyen de comptage (2) sur un état de donnée et un moyen (CK sur 7) qui répond à une impulsion de stimulation artificielle en positionnant ladite bascule (7) afin de valider ledit premier moyen logique de type porte (3), et

un deuxième moyen (15) qui répond à ladite mémoire (13), dans le cas où une fonction d'hystérésis de vitesse est souhaitée, et à un signal de battement cardiaque survenant naturellement en repositionnant ladite bascule (7) de façon à valider ledit deuxième moyen logique du type porte (4).

5. Stimulateur d'impulsions cardiaque à la demande selon l'une quelconque des revendications 1 à 4, caractérisé en ce que ledit moyen de comptage (2) est conçu pour fournir des impulsions de sortie à plus d'un premier et un deuxième taux de comptage, lesdits taux supplémentaires correspondant à des taux d'impulsions de stimulation prédéterminés respectifs, ladite mémoire (13) comprend en outre un moyen (17) permettant de sélectionner l'un desdits taux supplémentaires, et ledit moyen de commutation comporte des moyens ($3^1$—$3^4$, $4^1$—$4^4$, $5^1$—$5^4$, 16) qui répond à ladite mémoire (13) et audit moyen logique en délivrant des impulsions de l'un quelconque donné des taux d'impulsions audit amplificateur de sortie (6).

### Patentansprüche

1. Bedarfsherzschrittmacher mit einer Raten-Hysteresefunktion, welche mittels getrennter Programmiermittel programmierbar gespeichert werden kann, so daß dieser Betriebszustand nach Wunsch einstellbar ist, mit:

a) einem Oszillator (1), welcher mit einer vorgegebenen Rate arbeitet,

b) einem Ausgangsverstärker (6) zum Erzeugen von künstlichen Stimulationsimpulsen,

c) einem Eingagsverstärker (11) zum Aufnehmen von auftretenden natürlichen Herzschlagsignalen,

d) Mitteln, welche einen Speicher (13) umfassen, der durch die äußeren Programmiermittel programmierbar ist, um den Schrittmacher gegebenenfalls in seinem Hysterese-Betriebszustand zu betreiben,

dadurch gekennzeichnet, daß der Schrittmacher enthält:

e) Zählmittel (2), welche von Oszillator (1) beeinflußt werden, um Ausgangsimpulse mit wenigstens zwei verschiedenen Zählraten (Qy, Qx) abzugeben,

f) Schaltmittel (3, 4), welche alternativ auf die Zählraten hin ansprechen, um den Ausgangsverstärker (6) zu aktivieren und

g) logische Mittel (7, 15), welche von dem Speicher (13) und dem Eingangsverstärker (11) angesteuert werden, um die Schaltmittel (3, 4) zu beeinflussen, wodurch, wenn der Speicher (13) angibt, daß der Raten-Hysteresezustand nicht programmiert ist, der Ausgangsverstärker (6) Zählimpulse mit einer ersten Zählrate erhält und, wenn der Programmspeicher (13) angibt, daß der Hysteresezustand programmiert ist, der Ausgangsverstärker (6) mindestens einen Impuls mit der zweiten Impulsrate erhält, nachdem durch die logischen Mittel wenigstens ein natürlicher Herzschlag aufgenommen wurde.

2. Bedarfsherzschrittmacher nach Anspruch 1, dadurch gekennzeichnet, daß die logischen Schaltmittel ein Flip-Flop (7) enthalten, mit ersten und zweiten logischen Zuständen, welche die Schaltmittel (3, 4) ansteuern, um den Ausgangsverstärker (11) zum Empfang der Zählimpulse mit der ersten bzw. zweiten Rate zu aktivieren.

3. Bedarfsherzschrittmacher nach Anspruch 2, dadurch gekennzeichnet, daß die Schaltmittel erste (3) bzw. zweite (4) Tormittel enthalten, welche Ausgangsimpulse mit ersten und zweiten Raten aufnehmen, wobei die Tormittel in komplementärer Weise durch die ersten und zweiten logischen Zustände des Flip-Flops (7) aktiviert werden.

4. Bedarfsherzschrittmacher nach Anspruch 3, dadurch gekennzeichnet, daß die logischen Schaltmittel umfassen: erste Mittel, welche Mittel (8, 9) enthalten, die auf künstliche Stimulationsimpulse oder natürlich auftretende Herzsignale ansprechen, um die Zählmittel (2) in einen Datenzustand zurückzusetzen und Mittel (CK bei 7), welche auf künstliche Stimulationsimpulse ansprechen, um das Flip-Flop (7) derart zu setzen, daß die ersten Tormittel (3) aktiviert werden sowie zweite Mittel (15), welche auf die Speichermittel (13) ansprechen, falls die Raten-Hysteresefunktion aktiviert ist und auf ein natürlich auftretendes Herzsignal, um das Flip-Flop (7) zurückzusetzen, so daß die zweiten Tormittel (4) aktiviert sind.

5. Bedarfsherzschrittmacher nach einem der Ansprüche 1 bis 4, dadurch gekennzeichnet, daß die Zählmittel (2) derart ausgebildet sind, daß sie Ausgangsimpulse mit mehr als der ersten und zweiten Impulsrate abgeben Können, wobei die zusätzlichen Raten jeweils vorgegebenen Stimulationsimpulsraten entsprechen, und der Speicher (13) zusätzliche Mittel (17) umfaßt, um eine oder mehrere zusätzliche Raten auszuwählen, wobei die Schaltmittel weitere Mittel ($3^1$—$3^3$, $4^1$—$4^4$, $5^1$—$5^4$, 16) umfassen, welche vom Speicher (13) und zusätzlichen logischen Mitteln angesteuert werden, um Impulse mit jeder der vorgegebenen Impulsraten an den Ausgangsverstärker (6) abzugeben.

FIG.1.

**0 000 987**

Natural beats (a)

Pacemaker pulses (b)

Qy          Qy

Natural beats (c)

Pacemaker pulses (d)

Qy          Qx    Qy          Qx    Qy

Flip Flop    1
7    (e)
0

Note: (a) and (b) are with no hysteresis function,
(c),(d) and (e) are with hysteresis function.

# FIG.2.

2

**0 000 987**

FIG.3.